(19) Europäisches Patentamt / European Patent Office / Office européen des brevets

(11) **EP 4 763 245 A1**

(12) **EUROPEAN PATENT APPLICATION**

(43) Date of publication:
24.06.2026 Bulletin 2026/26

(21) Application number: 25224875.2

(22) Date of filing: **18.12.2025**

(51) International Patent Classification (IPC):
**A61M 1/36** (2006.01)     **A61M 1/38** (2006.01)
**G01N 33/49** (2006.01)

(52) Cooperative Patent Classification (CPC):
**A61M 1/3696; A61M 1/38;** A61M 2202/0427;
A61M 2205/3306; A61M 2205/52; G01N 33/491

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **20.12.2024 US 202463736651 P**

(71) Applicant: **Fenwal, Inc.**
**Lake Zurich, IL 60047 (US)**

(72) Inventors:
• **KUSTERS, Benjamin E.**
**Lake Zurich, 60047 (US)**
• **MIN, Kyungyoon**
**Lake Zurich, 60047 (US)**

(74) Representative: **Maikowski & Ninnemann**
**Patentanwälte Partnerschaft mbB**
**Postfach 15 09 20**
**10671 Berlin (DE)**

(54) **ADJUSTMENT OF THE MEASUREMENT OF THE CELL CONCENTRATION OF A FLUID**

(57)   A biological fluid processing device includes pump and valve systems, a controller, and an optical detection assembly. During a biological fluid processing procedure, the controller controls the operation of the pump and valve systems to separate a cell-containing fluid from the blood of a blood source and controls the optical detection assembly to emit light through the cell-containing fluid. The controller receives signals from the optical detection assembly that are indicative of the intensity of light that has passed through the cell-containing fluid. The controller receives or calculates an adjustment factor that is derived from historical data reflecting a cell collection procedure previously executed for the same blood source in which the same type of cell was collected. The controller determines an adjusted cell concentration of the cell-containing fluid based at least in part on the signals from the light detector and the adjustment factor.

FIG. 1

EP 4 763 245 A1

**Description**

Background

Field of the Disclosure

The present disclosure relates to determination of the cellular concentration of a fluid. More particularly, the present disclosure relates to adjustment of the measurement of the cellular concentration of a fluid determined by a sensor.

Description of Related Art

Various blood processing systems now make it possible to collect particular blood constituents, instead of whole blood, from a blood source. Typically, in such systems, whole blood is drawn from a blood source, the particular blood component or constituent is separated, removed, and collected, and the remaining blood constituents are returned to the blood source.

According to one approach, whole blood may be separated into red blood cells and platelet-rich plasma, with the platelet-rich plasma subsequently being separated into platelet concentrate and platelet-poor plasma. In such an example, the platelet concentrate may be collected as a platelet product and the platelet-poor plasma may be collected in another container as a plasma product or may be returned to the blood source.

According to one conventional approach, an optical detection assembly is employed during a platelet separation and collection procedure to determine the platelet concentration of one of the fluids (e.g., of the platelet-rich plasma). A typical optical detection assembly includes a light source (e.g., a laser or a light-emitting diode) configured to emit light into a fluid-containing vessel of a fluid flow circuit, with a light detector (e.g., a photodiode) configured to receive light exiting the vessel. The light detector transmits a signal to a controller based upon the light it has received, with the controller using the signal to determine the instantaneous platelet concentration of the fluid. Signals may be periodically or continuously sent from the light detector to the controller to keep the controller apprised of the platelet concentration of the fluid throughout the procedure. The controller may use the measured platelet concentration(s) in combination with the measured volume of platelet product that has been collected to calculate an estimated platelet yield during and at the end of the procedure.

After a platelet collection procedure has been completed, the collected platelet product may be analyzed (e.g., using a cell counter) to calculate an actual platelet yield. It has been found (by comparing the estimated platelet yield calculated by the controller to the actual platelet yield determined by the cell counter) that the estimated platelet yield calculated by the controller may differ from the actual platelet yield. It would, thus, be advantageous to improve the operation of the controller so that the estimated platelet yield is closer to the actual platelet yield of the collected platelet product.

Summary

There are several aspects of the present subject matter which may be embodied separately or together in the devices and methods described and claimed below. These aspects may be employed alone or in combination with other aspects of the subject matter described herein, and the description of these aspects together is not intended to preclude the use of these aspects separately or the claiming of such aspects separately as set forth in the claims appended hereto.

In one aspect, a biological fluid processing device includes a pump system, a valve system, a controller programmed to control the operation of the pump system and the valve system to execute a biological fluid processing procedure for a blood source, and an optical detection assembly. The optical detection assembly includes a light source configured and oriented to emit a light into a cell-containing fluid in a vessel during the biological fluid processing procedure for the blood source and a light detector configured to receive at least a portion of the light exiting the vessel. The controller is further programmed to receive a signal from the light detector during the biological fluid processing procedure for the blood source, with the signal from the light detector being indicative of an intensity of the light received by the light detector. The controller receives or calculates an adjustment factor derived from historical data reflecting a cell collection procedure previously executed for the blood source in which the same type of cell was collected. The controller then determines an adjusted cell concentration of the cell-containing fluid based at least in part on the signal from the light detector and the adjustment factor.

In another aspect, a biological fluid processing system includes a biological fluid processing device having a pump system, a valve system, a controller programmed to control the operation of the pump system and the valve system to execute a biological fluid processing procedure for a blood source, and an optical detection assembly. The optical detection assembly includes a light source configured and oriented to emit a light into a cell-containing fluid in a vessel during the biological fluid processing procedure for the blood source and a light detector configured to receive at least a portion of the light exiting the vessel. The biological fluid processing system also includes a data management system programmed to store historical data reflecting a cell collection procedure previously executed for the blood source in which the same type of cell was collected. The controller is further programmed to receive a signal from the light detector during the biological fluid processing procedure for the blood source, with the signal from the light detector being indicative of an intensity of the light received by the light detector. The controller receives from the data management system an adjustment factor derived from the historical data or receives the historical data from the data management system and calculates the adjustment factor. The controller then determines an adjusted cell concentration of the cell-containing fluid based at least in part on the signal from the light detector and the adjustment factor.

In yet another aspect, a controller-implemented method is provided for executing a biological fluid processing procedure for a blood source. The method (i.e. ex-vivo, non-surgical) includes separating a cell-containing fluid from blood from the blood source and emitting light through the cell-containing fluid. The method further includes receiving at least a portion of the light exiting the cell-containing fluid and generating a signal indicative of an intensity of the light exiting the cell-containing fluid. An adjustment factor is either received or calculated, with the adjustment factor derived from historical data reflecting a cell collection procedure previously executed for the blood source in which the same type of cell was collected. An adjusted cell concentration of the cell-containing fluid is then determined, based at least in part on the signal and the adjustment factor.

Brief Description of the Drawings

Fig. 1 is a perspective view of an exemplary hardware component of a biological fluid processing system according to an aspect of the present disclosure;

Fig. 2 is a schematic view of an exemplary disposable component that may be mounted to the hardware component of Fig. 1 to complete a biological fluid processing system according to an aspect of the present disclosure;

Fig. 3 is a perspective view of an exemplary optical detection assembly of the hardware component of Fig. 1, with a lid thereof in an open position;

Fig. 4 is a perspective view of the optical detection assembly of Fig. 3, with the lid in a closed position;

Fig. 5 is a perspective view of selected components of the optical detection assembly of Fig. 3; and

Fig. 6 is a flowchart illustrating an approach to adjusting the estimated platelet yield calculated by a controller of the hardware component of Fig. 1, according to an aspect of the present disclosure.

Description of the Illustrated Embodiments

The embodiments disclosed herein are for the purpose of providing an exemplary description of the present subject matter. They are, however, only exemplary, and the present subject matter may be embodied in various forms. Therefore, specific details disclosed herein are not to be interpreted as limiting the subject matter as defined in the accompanying claims.

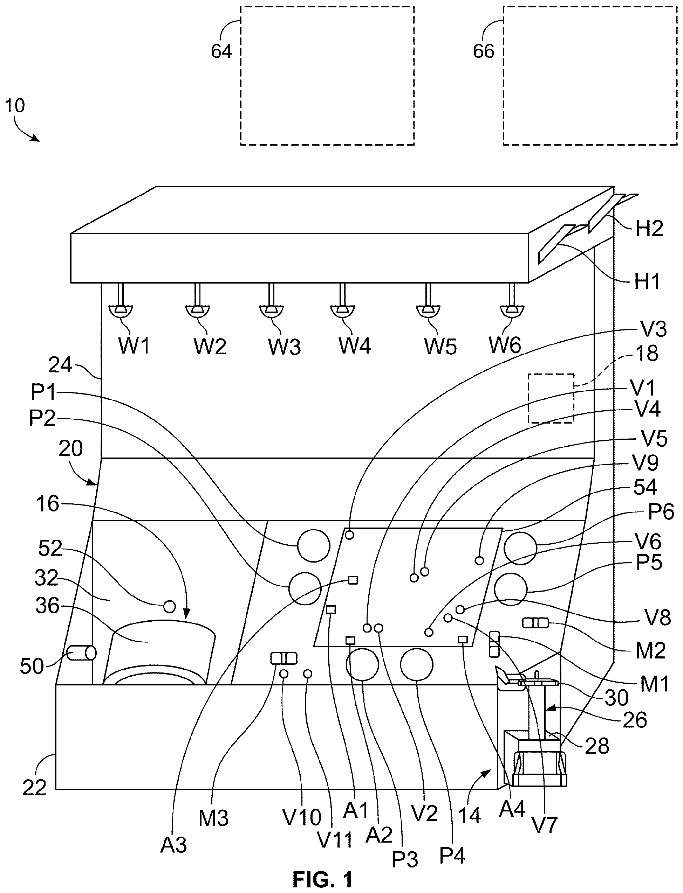

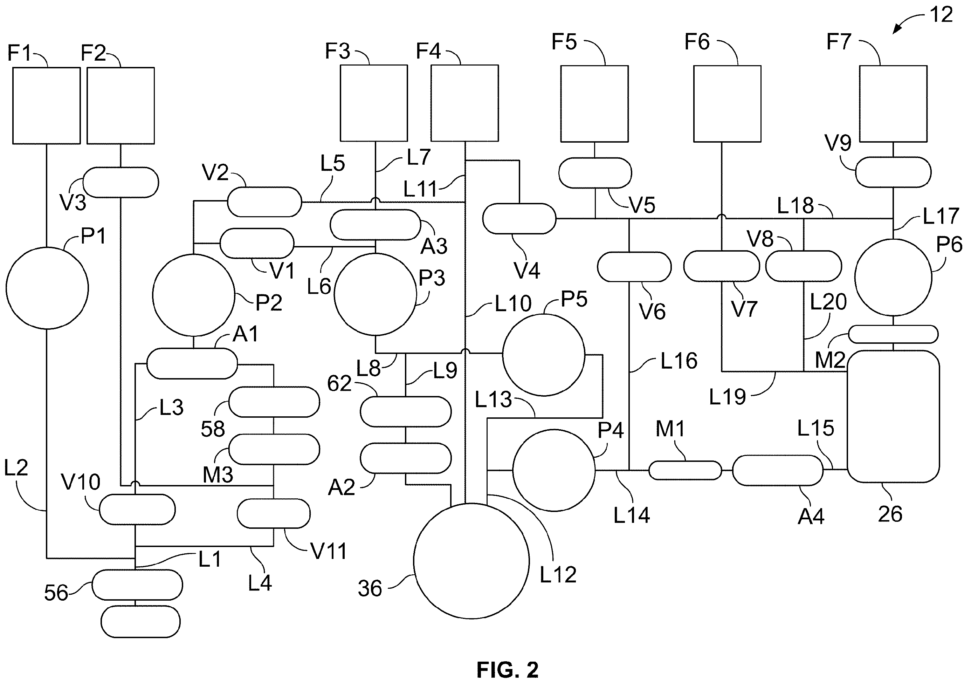

Figs. 1 and 2 show components of a biological fluid processing system that embodies various aspects of the present subject matter. Use of the system for separating blood into two or more components and collecting at least one of the components will be described herein, though it should be understood that systems according to the present disclosure can be used for processing a variety of different biological fluids.

Generally speaking, the system includes two principal components, a durable and reusable biological fluid processing device 10 (Fig. 1) and a disposable fluid flow circuit 12 (Fig. 2). The illustrated biological fluid processing device 10 includes a spinning membrane separator drive unit 14, a centrifuge or centrifugal separator 16, additional components that control fluid flow through the disposable fluid flow circuit 12, and a controller 18, which governs the operation of the other components of the biological fluid processing device 10 to perform a biological fluid processing procedure. While the principles described herein may be employed when using the biological fluid processing device 10 of Fig. 1, it should be understood that these same principles may be applied to other biological fluid processing devices, including devices employing single separation technologies or approaches.

I. The Durable Biological Fluid Processing Device

The biological fluid processing device 10 (Fig. 1) is configured as a durable item that is capable of long-term use. It should be understood that the biological fluid processing device 10 of Fig. 1 is merely exemplary of one possible configuration and that biological fluid processing devices according to the present disclosure may be differently configured.

In the illustrated embodiment, the biological fluid processing device 10 is embodied in a single housing or case 20. The illustrated case 20 includes a generally horizontal portion 22 (which may include an inclined or angled face or upper surface for enhanced visibility and ergonomics) and a generally vertical portion 24. The spinning membrane separator drive unit 14 and the centrifugal separator 16 are shown as being incorporated into the generally horizontal portion 22 of the case 20, while the controller 18 is shown as being incorporated into the generally vertical portion 24.

A. Spinning Membrane Separator Drive Unit

The biological fluid processing device 10 includes a spinner support or spinning membrane separator drive unit 14 for accommodating a generally cylindrical spinning membrane separator 26 of the fluid flow circuit 12. U.S. Patent No. 5,194,145 (which is hereby incorporated herein by reference) describes an exemplary spinning membrane separator drive unit that would be suitable for incorporation into the fluid processing device 10, but it should be understood that the spinning membrane separator drive unit 14 may be differently configured without departing from the scope of the present disclosure.

The illustrated spinning membrane separator drive unit 14 has a base 28 configured to receive a lower portion of the spinning membrane separator 26 and an upper end cap 30 to receive an upper portion of the spinning membrane separator 26. Preferably, the upper end cap 30 is positioned directly above the base 28 to orient a spinning membrane separator 26 received by the spinning membrane separator drive unit 14 vertically and to define a vertical axis about which the spinning membrane separator 26 is spun. While it may be advantageous for the spinning membrane separator drive unit 14 to vertically orient a spinning membrane separator 26, it is also within the scope of the present disclosure for the spinning membrane separator 26 to be differently oriented when mounted to the biological fluid processing device 10.

In one embodiment, one of the base 28 and upper end cap 30 of the spinning membrane separator drive unit 14 is movable with respect to the other, which may allow differently sized spinning membrane separators 26 to be received by the spinning membrane separator drive unit 14. For example, the upper end cap 30 may be translated vertically with respect to the base 28 and locked in a plurality of different positions, with each locking position corresponding to a differently sized spinning membrane separator 26.

At least one of the base 28 and the upper end cap 30 is configured to spin one or more components of the spinning membrane separator 26 about the axis defined by the spinning membrane separator drive unit 14. The mechanism by which the spinning membrane separator drive unit 14 spins one or more components of the spinning membrane separator 26 may vary without departing from the scope of the present disclosure. In one embodiment, a component of the spinning membrane separator 26 to be spun includes at least one element configured to be acted upon by a magnet (e.g., a metallic material), while the spinning membrane separator drive unit 14 includes a magnet (e.g., a series of magnetic coils or semicircular arcs). By modulating the magnetic field acting upon the aforementioned element of the spinning membrane separator 26, the component or components of the spinning membrane separator 26 may be made to spin in different directions and at varying speeds. In other embodiments, different mechanisms may be employed to spin the component or components of the spinning membrane separator 26.

Regardless of the mechanism by which the spinning membrane separator drive unit 14 spins the component or components of the spinning membrane separator 26, the component or components of the spinning membrane separator 26 is/are preferably spun at a speed that is sufficient to create Taylor vortices in a gap between the spinning component and a stationary component of the spinning membrane separator 26 (or a component that spins at a different speed). Fluid to be separated within the spinning membrane separator 26 flows through this gap, and filtration may be dramatically improved by the creation of Taylor vortices.

B. Centrifugal Separator

As for the centrifugal separator 16, it includes a centrifuge compartment 32 that receives a centrifugal separation chamber 36 of the fluid flow circuit 12, as well as other components of the centrifugal separator 16. Further details as to the centrifugal separator are set forth in PCT Patent Application Publication No. WO 2018/053217 A1, which is hereby incorporated herein by reference.

Fluid (e.g., anticoagulated whole blood) is introduced into the centrifugal separation chamber 36 by an umbilicus, with the fluid being separated into a layer of less dense components (e.g., platelet-rich plasma, in the case of blood being separated) and a layer of more dense components (e.g., packed red blood cells) within the centrifugal separation chamber 36 as a result of centrifugal forces as it rotates. Components of an interface monitoring system may be positioned within the centrifuge compartment 32 to oversee separation of fluid within the centrifugal separation chamber 36. The interface monitoring system may include a light source 50 and a light detector 52, which is positioned and oriented to receive at least a portion of the light emitted by the light source 50.

The orientation of the various components of the interface monitoring system depends at least in part on the particular configuration of the centrifugal separation chamber 36. In general, though, the light source 50 emits a light beam (e.g., a laser light beam) through the separated fluid components within the centrifugal separation chamber 36 (which may be formed of a material that substantially transmits the light or at least a particular wavelength of the light without absorbing it). A portion of the light reaches the light detector 52, which transmits a signal to the controller 18 that is indicative of the location of an interface between the separated fluid components. If the controller 18 determines that the interface is in the wrong location (which can affect the separation efficiency of the centrifugal separator 16 and/or the quality of the separated fluid components), then it can issue commands to the appropriate components of the biological fluid processing device 10 to modify their operation so as to move the interface to the proper location.

C. Other Components Of The Biological Fluid Processing Device

In addition to the spinning membrane separator drive unit 14 and the centrifugal separator 16, the biological fluid processing device 10 may include other components compactly arranged to aid fluid processing.

The generally horizontal portion 22 of the case 20 of the illustrated fluid processing device 10 includes a cassette station 54, which accommodates a flow control cassette of the fluid flow circuit 12. In one embodiment, the cassette station 54 is similarly configured to the cassette station of U.S. Patent No. 5,868,696 (which is hereby incorporated herein by reference), but is adapted to include additional components and functionality. The illustrated cassette station 54 includes a plurality of clamps or valves V1-V9 (which are collectively referred to herein as the "valve system" of the biological fluid processing system 10), which move between a plurality of positions (e.g., between a retracted or lowered position and an actuated or raised position) to selectively contact or otherwise interact with corresponding valve stations of the flow control cassette of the fluid flow circuit 12. Depending on the configuration of the fluid flow circuit 12, its cassette may not include a valve station for each valve V1-V9 of the cassette station 54, in which case fewer than all of the valves V1-V9 will be used in a fluid processing procedure.

In the actuated position, a valve V1-V9 engages the associated valve station to prevent fluid flow through that valve station (e.g., by closing one or more ports associated with the valve station, thereby preventing fluid flow through that port or ports). In the retracted position, a valve V1-V9 is disengaged from the associated valve station (or less forcefully contacts the associated valve station than when in the actuated position) to allow fluid flow through that valve station (e.g., by opening one or more ports associated with the valve station, thereby allowing fluid flow through that port or ports). Additional clamps or valves V10 and V11 of the valve system may be positioned outside of the cassette station 54 to interact with portions of valve stations (which may be lengths of tubing) of the fluid flow circuit 12 to selectively allow and prevent fluid flow therethrough. The valves V1-V9 and corresponding valve stations of the cassette station 54 and cassette may be differently configured and operate differently from the valves V10 and V11 and the valve stations that are spaced away from the cassette station 54.

The cassette station 54 may be provided with additional components, such as pressure sensors A1-A4, which interact with sensor stations of the cassette to monitor the pressure at various locations of the fluid flow circuit 12. For example, if the fluid source is a human donor, one or more of the pressure sensors A1-A4 may be configured to monitor the pressure of the donor's vein during blood draw and return. Other pressure sensors A1-A4 may monitor the pressure of the spinning membrane separator 26 and the centrifugal separation chamber 36. The controller 18 may receive signals from the pressure sensors A1-A4 that are indicative of the pressure within the fluid flow circuit 12 and, if a signal indicates a low- or highpressure condition, the controller 18 may initiate an alarm or error condition to alert an operator to the condition and/or to attempt to bring the pressure to an acceptable level without operator intervention.

The biological fluid processing device 10 may also include a plurality of pumps P1-P6 (which are collectively referred to herein as the "pump system" of the biological fluid processing device 10) to cause fluid to flow through the fluid flow circuit 12. The pumps P1-P6 may be differently or similarly configured and/or function similarly or differently from each other. In the illustrated embodiment, the pumps P1-P6 are configured as peristaltic pumps, which may be generally configured as described in U.S. Patent No. 5,868,696. Each pump P1-P6 engages a different tubing loop extending from a side surface of the flow control cassette and may be selectively operated under command of the controller 18 to cause fluid to flow through a portion of the fluid flow circuit 12. In one embodiment, all or a portion of the cassette station 54 may be capable of translational motion in and out of the case 20 to allow for automatic loading of the tubing loops into the associated pump P1-P6. In another exemplary embodiment, rather than employing peristaltic pumps, pneumatic pumps may be employed, with actuators incorporated into the cassette station 54 interacting with suitably configured portions of the fluid flow circuit 12 (e.g., pump stations of a cassette mounted to the cassette station 54) to convey fluid through the fluid flow circuit 12.

The illustrated biological fluid processing device 10 also includes a spinner inlet sensor M1 for determining one or more properties of a fluid flowing into a spinning membrane separator 26 mounted within the spinning membrane separator drive unit 14. If the fluid flowing into the spinning membrane separator 26 is whole blood (which may include anticoagulated whole blood), the spinner inlet sensor M1 may be configured to determine the hematocrit of the blood flowing into the spinning membrane separator 26. If the fluid flowing into the spinning membrane separator 26 is platelet-rich plasma, the spinner inlet sensor M1 may be configured to determine the platelet concentration of platelet-rich plasma flowing into the spinning membrane separator 26. The spinner inlet sensor M1 may detect the one or more properties of a fluid by optically monitoring the fluid as it flows through tubing of the fluid flow circuit 12, or by any other suitable approach.

The controller 18 may receive signals from the spinner inlet sensor M1 that are indicative of the one or more properties of fluid flowing into the spinning membrane separator 26 and use the signals to optimize the fluid processing procedure based upon that property or properties. If the property or properties is/are outside of an acceptable range, then the controller 18 may initiate an alarm or error condition to alert an operator to the condition. A suitable device and method for monitoring hematocrit and/or platelet concentration is described in U.S. Patent No. 6,419,822 (which is hereby incorporated herein by reference), but it should be understood that a different approach may also be employed for monitoring one or more properties of a fluid or fluid component flowing into the spinning membrane separator 26.

The illustrated biological fluid processing device 10 further includes a spinner outlet sensor M2, which accommodates tubing of the fluid flow circuit 12 that flows a separated fluid component out of the spinning membrane separator 26. The spinner outlet sensor M2 monitors the separated fluid component to determine one or more properties thereof, and may do so by optically monitoring the separated fluid component as it flows through the tubing or by any other suitable approach. In one embodiment, separated plasma flows through the tubing, in which case the spinner outlet sensor M2 may be configured to determine the amount of cellular blood components in the plasma and/or whether the plasma is hemolytic and/or lipemic. This may be done using an optical monitor of the type described in U.S. Patent No. 8,556,793 (which is hereby incorporated herein by reference) that measures the optical density of the fluid in the associated tubing, or by any other suitable device and/or method.

The illustrated fluid processing device also includes an air detector M3 (e.g., an ultrasonic bubble detector), which accommodates tubing of the fluid flow circuit 12 that flows fluid to a recipient. It may be advantageous to prevent air from reaching the recipient, whether a human recipient (e.g., the same human that serves as the blood source) or a non-human recipient (e.g., a storage bag or container), so the air detector M3 may transmit signals to the controller 18 that are indicative of the presence or absence of air in the tubing. If the signal is indicative of air being present in the tubing, the controller 18 may initiate an alarm or error condition to alert an operator to the condition and/or to take corrective action to prevent the air from reaching the recipient (e.g., by reversing the flow of fluid through the tubing or diverting flow to a vent location).

The generally vertical portion 24 of the case 20 may include a plurality of volume measurement systems W1-W6 (six are shown, but more or fewer may be provided), each configured to be associated with one or more fluid containers F1-F7 of the fluid flow circuit 12 (Fig. 2). Each volume measurement system W1-W6 is configured to work in combination with the controller 18 to measure a current volume of fluid within an associated fluid container F1-F7 and to calculate a change in that volume between two or more points in time. The individual volume measurement systems W1-W6 may be variously configured without departing from the scope of the present disclosure, which may include two or more of the volume measurement systems W1-W6 being differently configured. In one exemplary embodiment, a volume measurement system W1-W6 may be configured as or include a weight scale configured to support and measure the weight of a fluid within an associated fluid container F1-F7 (with the measured weight being converted to a volume by a component of the volume measurement system W1-W6 or by the controller 18). In another exemplary embodiment, a volume measurement system W1-W6 may include one or more sensors configured to detect a volume and/or a change in volume of a fluid within an associated fluid container F1-F7. Volume measurement systems including additional components (e.g., both a weight scale and a sensor) and/or alternative components may also be employed without departing from the scope of the present disclosure.

Regardless of its particular configuration, each volume measurement system W1-W6 transmits to the controller 18 a signal that is indicative of the volume of the fluid within the associated container F1-F7 to track the change of volume during the course of a procedure. This allows the controller 18 to process the incremental volume changes to derive fluid processing volumes and flow rates and subsequently generate signals to control processing events based, at least in part, upon the derived processing volumes. For example, the controller 18 may diagnose leaks and obstructions in the fluid flow circuit 12 and alert an operator.

The illustrated case 20 is also provided with a plurality of hooks or supports H1 and H2 that may support various components of the fluid flow circuit 12 or other suitably sized and configured objects.

D. Controller

According to an aspect of the present disclosure, the biological fluid processing device 10 includes a controller 18, which is suitably configured and/or programmed to control operation of the biological fluid processing device 10. In one embodiment, the controller 18 comprises a main processing unit (MPU), which can comprise, e.g., a Pentium™ type microprocessor made by Intel Corporation, although other types of conventional microprocessors can be used. In one embodiment, the controller 18 may be mounted inside the generally vertical portion 24 of the case 20, adjacent to or incorporated into an operator interface station (e.g., a touchscreen). In other embodiments, the controller 18 and operator interface station may be associated with the generally horizontal portion 22 or may be incorporated into a separate device that is connected (either physically, by a cable or the like, or wirelessly) to the biological fluid processing device 10.

The controller 18 is configured and/or programmed to execute at least one biological fluid processing procedure but, more advantageously, is configured and/or programmed to execute a variety of different biological fluid processing procedures. For example, the controller 18 may be configured and/or programmed to carry out one or more of the following: a double unit red blood cell collection procedure, a plasma collection procedure, a plasma/red blood cell collection procedure, a red blood cell/platelet/plasma collection procedure, a platelet collection procedure, and a platelet/plasma collection procedure.

More particularly, in carrying out these fluid processing procedures, the controller 18 is configured and/or programmed to control one or more of the following tasks: drawing fluid into a fluid flow circuit 12 mounted to the biological fluid processing device 10, conveying fluid through the fluid flow circuit 12 to a location for separation (i.e., into a spinning membrane separator 26 or centrifugal separation chamber 36 of the fluid flow circuit 12), separating the fluid into two or more components as desired, and conveying the separated components into storage containers, to a second location for further separation (e.g., into whichever of the spinning membrane separator 26 and centrifugal separation chamber 36 that was not used in the initial separation stage), or to a recipient (which may be a source from which the fluid was originally drawn).

This may include instructing the spinning membrane separator drive unit 14 and/or the centrifugal separator 16 to operate at a particular rotational speed and instructing a pump P1-P6 to convey fluid through a portion of the fluid flow circuit 12 at a particular flow rate. Hence, while it may be described herein that a particular component of the biological fluid processing device 10 (e.g., the spinning membrane separator drive unit 14 or the centrifugal separator 16) performs a particular function, it should be understood that that component is being controlled by the controller 18 to perform that function.

Before, during, and after a procedure, the controller 18 may receive signals from various components of the biological fluid processing device 10 (e.g., the pressure sensors A1-A4) to monitor various aspects of the operation of the biological fluid processing device 10 and characteristics of the fluid and separated fluid components as they flow through the fluid flow circuit 12. If the operation of any of the components and/or one or more characteristics of the fluid or separated fluid components is outside of an acceptable range, then the controller 18 may initiate an alarm or error condition to alert the operator and/or take action to attempt to correct the condition. The appropriate corrective action will depend upon the particular error condition and may include action that is carried out with or without the involvement of an operator.

For example, the controller 18 may include an interface control module, which receives signals from the light detector 52 of the interface monitoring system. The signals that the controller 18 receives from the light detector 52 are indicative of the location of an interface between the separated fluid components within the centrifugal separation chamber 36. If the controller 18 determines that the interface is in the wrong location, then it can issue commands to the appropriate components of the biological fluid processing device 10 to modify their operation so as to move the interface to the proper location. For example, the controller 18 may instruct one of the pumps P1-P6 to cause fluid to flow into the centrifugal separation chamber 36 at a different rate and/or for a separated fluid component to be removed from the centrifugal separation chamber 36 at a different rate and/or for the centrifugal separation chamber 36 to be spun at a different speed by the centrifugal separator 16.

If provided, an operator interface station associated with the controller 18 allows the operator to view on a screen or display (in alpha-numeric format and/or as graphical images) information regarding the operation of the system. The operator interface station also allows the operator to select applications to be executed by the controller 18, as well as to change certain functions and performance criteria of the system. If configured as a touchscreen, the screen of the operator interface station can receive input from an operator via touch-activation. Otherwise, if the screen is not a touchscreen, then the operator interface station may receive input from an operator via a separate input device, such as a computer mouse or keyboard. It is also within the scope of the present disclosure for the operator interface station to receive input from both a touchscreen and a separate input device, such as a keypad.

II. The Disposable Fluid Flow Circuit

As for the fluid flow circuit or flow set 12 (Fig. 2), it is intended to be a sterile, single use, disposable item. Before beginning a given fluid processing procedure, the operator loads various components of the fluid flow circuit 12 in the case 20 in association with the biological fluid processing device 10. The controller 18 implements the procedure based upon preset protocols, taking into account other input from the operator. Upon completing the procedure, the operator removes the fluid flow circuit 12 from association with the biological fluid processing device 10. The portions of the fluid flow circuit 12 holding the collected fluid component or components (e.g., collection containers or bags) are removed from the case 20 and retained for storage, transfusion, or further processing. The remainder of the fluid flow circuit 12 is removed from the case 20 and discarded.

In the illustrated embodiment, the fluid flow circuit 12 includes a cassette, to which the other components of the fluid flow circuit 12 are connected by flexible tubing. The other components may include a plurality of fluid containers F1-F7. In the context of the present disclosure these containers include an anticoagulant container F1, a saline container F2, an in-process container F3, a return container F4, a plasma collection container F5, a platelet collection container F6, and an (optional) additive container F7. The illustrated flow circuit 12 further includes one or more fluid source access devices (e.g., a connector for accessing blood within a fluid container or a phlebotomy needle), a spinning membrane separator 26 and a centrifugal separation chamber 36.

The flow control cassette provides a centralized, programmable, integrated platform for all the pumping and many of the valving functions required for a given fluid processing procedure. In one embodiment, the cassette is similarly configured to the cassette of U.S. Patent No. 5,868,696, but is adapted to include additional components (e.g., more tubing loops) and functionality.

In use, the cassette is mounted to the cassette station 54 of the biological fluid processing device 10 so as to align each sensor station with an associated pressure sensor A1-A4 of the cassette station 54 and its valve stations with an associated valve V1-V9. Each valve station may define one or more ports that allow fluid communication between the valve station and another interior cavity of the cassette (e.g., a flow path). As described above, each valve V1-V9 is movable under command of the controller 18 to move between a plurality of positions (e.g., between a retracted or lowered position and an actuated or raised position) to selectively contact the valve stations of the cassette. In the actuated position, a valve V1-V9 engages the associated valve station to close one or more of its ports to prevent fluid flow therethrough. In the retracted position, a valve V1-V9 is disengaged from the associated valve station (or less forcefully contacts the associated valve station than when in the actuated position) to open one or more ports associated with the valve station, thereby allowing fluid flow therethrough.

A plurality of tubing loops extend from the side surface of the cassette to interact with pumps P1-P6 of the biological fluid processing device 10. The different pumps P1-P6 may interact with the tubing loops of the cassette to perform different tasks during a procedure, but in the context of the present disclosure, a different one of the pumps P1-P6 may be configured to serve as an anticoagulant pump P1, a source pump P2, a centrifuge pump P3, an outlet pump P4, a recirculation pump P5, and a plasma pump P6. If the pumps P1-P6 are differently configured (e.g., if they are configured as pneumatic pumps), then the cassette may be differently configured (e.g., with pump stations aligned with pneumatic pump actuators) to allow for the pumps P1-P6 to convey fluid through the cassette.

Additional tubing extends from the side surface of the cassette to connect to the other components of the fluid flow circuit 12, such as the various fluid containers F1-F7, the spinning membrane separator 26, and the centrifugal separation chamber 36. The tubing connected to the centrifugal separator chamber 36 (which includes one inlet tube and two outlet tubes) may be aggregated into an umbilicus.

Various additional components may be incorporated into the tubing leading out of the cassette or into one of the cavities of the cassette. For example, a manual clamp 56 may be associated with a line or lines leading to the fluid source, a return line filter 58 (e.g., a microaggregate filter) may be associated with a line leading to a fluid recipient, and/or an air trap 62 may be positioned on a line upstream of the centrifugal separation chamber 36.

III. Exemplary Biological Fluid Processing Procedure

An exemplary biological fluid processing procedure according to the present disclosure will now be described.

Prior to processing, an operator selects the desired protocol (e.g., using an operator interface station, if provided), which informs the controller 18 of the manner in which it is to control the other components of the biological fluid processing device 10 during the procedure. This may include first selecting one of a plurality of possible procedures that the system is capable of executing and then, after selecting the nature of the procedure, selecting one or more parameters to be in effect during the procedure. For example, this may include selecting a platelet collection procedure from among a variety of blood separation procedures and then selecting a total volume of blood to be processed or a target volume of platelets to be collected during the procedure. If the fluid source is a living source (e.g., a donor or patient), the operator may proceed to enter various parameters, such as the sex/height/weight of the source. In one embodiment, the operator may also enter one or more characteristics of the fluid to be processed, such as a platelet pre-count.

Once the controller 18 has received the necessary input, it may proceed to instruct the operator to mount the fluid flow circuit 12 to the biological fluid processing device 10. If there are any fluid containers (e.g., a platelet additive solution container) that are not integrally formed with the fluid flow circuit 12, they may be connected to the fluid flow circuit 12 (e.g., by piercing a septum of a tube of the fluid flow circuit 12 or via a luer connector), with the fluid flow circuit 12 then being mounted to the biological fluid processing device 10 (including the fluid containers F1-F7 being associated with the volume measurement systems W1-W6, as appropriate). In one exemplary embodiment, each volume measurement system W1-W6 includes a weight scale associated with a hook from which a fluid container may be hung. In another exemplary embodiment, at least one of the volume measurement systems W1-W6 includes a weight scale associated with a horizontal platform or surface, with a container being placed onto the platform or surface for support while the weight scale sends signals indicative of the weight of the container (and its contents) to be sent to the controller 18 throughout the course of a procedure. In other embodiments, a fluid container may be associated with a volume measurement system omitting a weight scale, but including other means for measuring the volume of fluid within the container (e.g., one or more sensors).

Once the fluid flow circuit 12 has been fully mounted to the biological fluid processing device 10, the controller 18 may proceed with an integrity check of the fluid flow circuit 12 to ensure that the various components of the fluid flow circuit 12 are properly connected and functioning. Following a successful integrity check, the fluid source is connected to the fluid flow circuit 12 (e.g., by connecting to a container of previously collected fluid or by phlebotomizing a donor), and the fluid flow circuit 12 may be primed (e.g., using saline pumped from a saline container F2 by operation of one or more of the pumps P1-P6 of the biological fluid processing device 10).

After the fluid flow circuit 12 has been primed, fluid processing may begin. In a first phase of an exemplary platelet collection procedure, blood is drawn into the fluid flow circuit 12 from a blood source. If the blood source is a donor, then blood may be drawn into the fluid flow circuit 12 through a single needle that is connected to the cassette by line L1. Line L1 may include a manual clamp 56 that may initially be in a closed position to prevent fluid flow through line L1. When processing is to begin, an operator may move the manual clamp 56 from its closed position to an open position to allow fluid flow through line L1.

The blood is drawn into line L1 by the source pump P2 of the biological fluid processing device 10. Anticoagulant from the anticoagulant container F1 may be drawn through line L2 under action of the anticoagulant pump P1 and added to the blood at a junction of lines L1 and L2.

In the illustrated embodiment, valve V10 is open to allow anticoagulated blood to flow through line L3 and a cassette sensor station associated with pressure sensor A1, while valve V11 is closed to prevent fluid flow through line L4. If the blood source is a living body (e.g., a donor), the pressure sensor A1 may communicate with the controller 18 to monitor the pressure within the vein of the blood source.

The cassette includes two valve stations downstream of the source pump P2, with valve V2 being closed to prevent flow through line L5 and valve V1 being open to allow flow through line L6. A portion of the blood is directed through line L7 and a cassette sensor station associated with pressure sensor A3 to the in-process container F3 and the remainder is directed through line L8 toward the centrifuge pump P3, which controls the amount of blood that is directed to the centrifugal separation chamber 36 instead of the in-process container F3. In particular, the flow rate of the source pump P2 is greater than the flow rate of the centrifuge pump P3, with the difference therebetween being equal to the flow rate of blood into the in-process container F3. The flow rates may be selected such that the in-process container F3 is partially or entirely filled with blood at the end of the draw phase.

The blood pumped through line L8 by the centrifuge pump P3 passes through line L19, an air trap 62, and a cassette sensor station associated with pressure sensor A2 (which works in combination with the controller 18 of the biological fluid processing device 10 to monitor the pressure in the centrifugal separation chamber 36) before reaching the centrifugal separation chamber 36 of the fluid flow circuit 12. The centrifugal separator 16 of the biological fluid processing device 10 manipulates the centrifugal separation chamber 36 to separate the blood in the centrifugal separation chamber 36 into platelet-rich plasma and packed red blood cells. In one embodiment, the centrifugal separation chamber 36 is rotated nominally at 4,500 rpm, but the particular rotational speed may vary depending on the flow rates of fluids into and out of the centrifugal separation chamber 36.

The packed red blood cells exit the centrifugal separation chamber 36 via line L10 and flow through line L11 into the return container F4. Platelet-rich plasma is drawn out of the centrifugal separation chamber 36 via line L12 by the combined operation of the recirculation and outlet pumps P5 and P4 of the biological fluid processing device 10. The platelet-rich plasma travels through line L12 until it reaches a junction, which splits into lines L13 and L14. The recirculation pump P5 is associated with line L13 and redirects a portion of the platelet-rich plasma to a junction, where it mixes with blood in line L8 that is being conveyed into the centrifugal separation chamber 36 by the centrifuge pump P3. Recirculating a portion of the platelet-rich plasma into the centrifugal separation chamber 36 with inflowing blood decreases the hematocrit of the blood entering the centrifugal separation chamber 36, which may improve separation efficiency. By such an arrangement, the flow rate of the fluid entering the centrifugal separation chamber 36 is equal to the sum of the flow rates of the centrifuge pump P3 and the recirculation pump P5. As the platelet-rich plasma drawn out of the centrifugal separation chamber 36 into line L13 by the recirculation pump P5 is immediately added back into the centrifugal separation chamber 36, the bulk or net platelet-rich plasma flow rate out of the centrifugal separation chamber 36 is equal to the flow rate of the outlet pump P4.

Line L14 ends at a junction, where it joins with lines L15 and L16. Valve V6 is closed to prevent fluid flow through line L16, thereby directing the separated platelet-rich plasma to the spinning membrane separator 26 via line L15. Before reaching the spinning membrane separator 26, the portion of the platelet-rich plasma conveyed through line L15 passes the spinner inlet sensor M1 and a cassette sensor station associated with pressure sensor A4. The spinner inlet sensor M1 may detect the concentration of platelets in the platelet-rich plasma entering the spinning membrane separator 26, while the pressure sensor A4 may monitor the pressure of the spinning membrane separator 26.

While valve V6 is typically closed, it may be selectively opened to divert all or a portion of the platelet-rich plasma from line L14 into and through line L16 and to the return container F4, if necessary. An example would be at the start of a procedure when separation is initializing and platelets are not yet exiting the centrifugal separation chamber 36, in which case the fluid conveyed through line L14 by the outlet pump P4 could be diverted to the return container F4.

The spinning membrane separator drive unit 14 of the biological fluid processing device 10 manipulates the spinning membrane separator 26 to separate the platelet-rich plasma into platelet-poor plasma ("plasma") and platelet concentrate ("platelets"). Plasma is pumped out of the spinning membrane separator 26 via line L17 by the plasma pump P6 of the biological fluid processing device 10. Valves V5, V6, V8, and V9 are closed to direct the separated plasma along line L18, through valve V4, and into the return container F4 (with the separated red blood cells). On the way to the return container F4, the plasma passes through spinner outlet sensor M2, which may cooperate with the controller 18 to determine one or more characteristics of the plasma, such as the amount of cellular blood components in the plasma and/or whether the plasma is hemolytic and/or lipemic.

The platelet concentrate is conveyed out of the spinning membrane separator 26 via line L19. There is no pump associated with line L19, so instead the flow rate at which the platelets exit the spinning membrane separator 26 is equal to the difference between the flow rates of the outlet pump P4 and plasma pump P6. Valve V8 is closed to prevent fluid flow through the line L20, thereby directing the flow of platelets along line L19, through valve V7, and into the platelet collection container F6. Valve V8 may be selectively opened to allow fluid flow through line L20 and to a junction, where it joins the plasma flowing through line L18 to the return container F4, if necessary.

Depending on the volume of platelets to be collected, the above-described draw stage may be repeated, with draw stages being alternated with return stages in which blood from the in-process container F3 is separated in the centrifugal separation chamber 36 while previously collected blood components in the return container F4 are returned to the blood source. During such return stages, the separated red blood cells and platelet-rich plasma may be variously routed through the fluid flow circuit 12, typically with an additional volume of platelets being collected in the platelet collection container F6 after being separated from platelet-poor plasma in the spinning membrane separator 26 (as during the draw stage). A platelet additive solution from the additive container F7 may be added to the collected platelets in the platelet collection container F6 before ending the procedure.


IV. Determination Of Instantaneous Cell Concentration And Estimated Cellular Yield

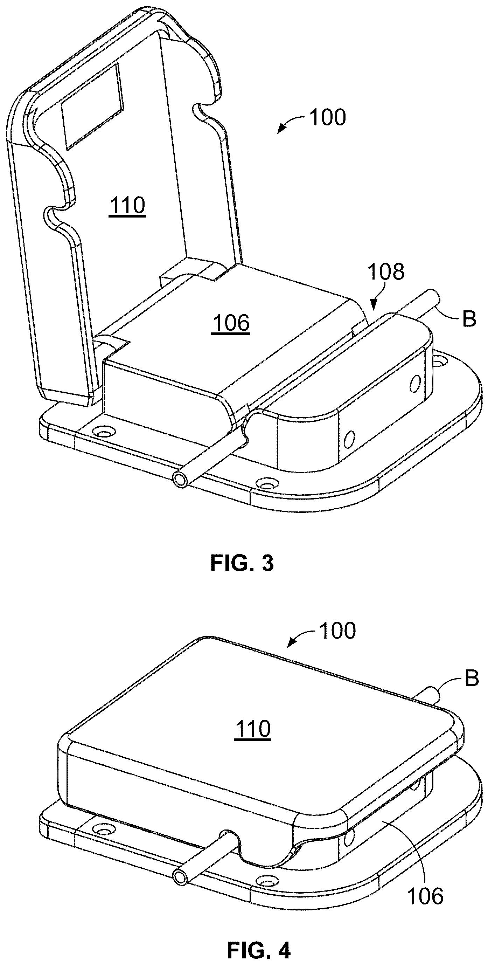

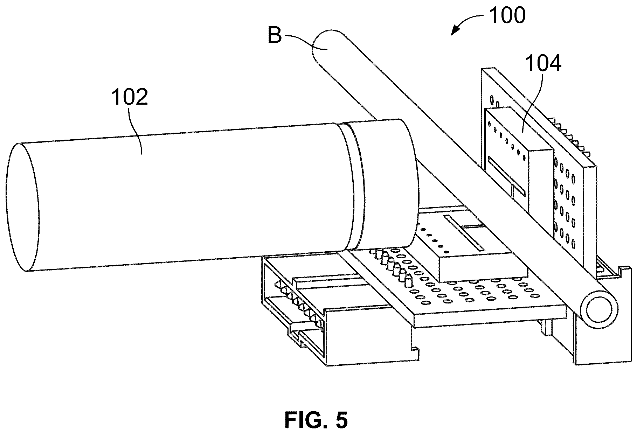

As noted above, the spinner inlet sensor M1 may be used in combination with the controller 18 to determine one or more properties of a fluid flowing into a spinning membrane separator 26, while the spinner outlet sensor M2 may be used to determine one or more properties of a fluid flowing out of the spinning membrane separator 26. Figs. 3-5 illustrate an exemplary optical detection assembly 100 that may be incorporated into the biological fluid processing device 10 to perform the functions of the spinner inlet sensor M1 or the spinner outlet sensor M2. In one embodiment, two such optical detection assemblies 100 may be incorporated into the biological fluid processing device 10, with one acting as the spinner inlet sensor M1 and the other acting as the spinner outlet sensor M2. While the optical detection assembly 100 of Figs. 3-5 will be described herein as being a component of the biological fluid processing device 10 of Fig. 1, it should be understood that optical detection assemblies according to the present disclosure may be incorporated into differently configured biological fluid processing devices or be provided as standalone devices that are not incorporated into a biological fluid processing device.

In the illustrated embodiment, the optical detection assembly 100 includes a light source 102 and a light detector array 104, which are spaced apart to accommodate a vessel "B" therebetween. When the optical detection assembly 100 is employed as a spinner inlet sensor M1, the vessel B may be line L15 of the fluid flow circuit 12, with the vessel B being line L17 of the fluid flow circuit 12 when the optical detection assembly 100 is instead employed as a spinner outlet sensor M2. It should be understood that the configuration of the vessel B used in combination with the optical detection assembly 100 may vary without departing from the scope of the present disclosure, provided that the vessel B is suitable for containing a fluid (which may include the vessel B being configured to accommodate the flow of a fluid therethrough) and formed of a material that is configured to transmit light emitted by the light source 102.

The illustrated optical detection assembly 100 includes a base 106 defining a slot or channel 108 configured to receive the vessel B. The channel 108 is configured to secure the vessel B in a desired orientation with respect to the light source 102 and the light detector array 104. The optical detection assembly 100 may also include a lid 110 (which is shown in Figs. 3 and 4 as being hingedly or pivotally associated to the base 106) to block external light from interfering with analysis of a fluid within the vessel B.

Light D emitted by the light source 102 (which may be variously configured without departing from the scope of the present disclosure) enters and then exits the vessel B after passing through a fluid within the vessel B. Light exiting a turbid media (such as blood or a blood component) will be dispersed, such that the light may be detected at multiple positions, rather than at a single location by a single light detector (e.g., an individual photodiode). It has been found that different fluids (e.g., ones having different cell concentrations) may result in emerging light beams having different dispersion patterns, with different individual light detectors of the light detector array 104 receiving different portions of the transmitted light. In general, the light detectors at the center of the light detector array 104 will tend to receive the most intense light, with the light detectors at each end of the light detector array 104 receiving little to no light.

A controller associated with the light detector array 104 (which may be the controller 18 of the biological fluid processing device 10 or a different, dedicated controller) receives signals from each of the individual light detectors of the light detector array 104, with each signal being indicative of the intensity of light received by the individual light detector that transmitted the signal to the controller. The collective set of signals received by the controller from the individual light detectors of the light detector array 104 is referred to herein as a "scattering profile," which may be understood as a graph or chart of the voltage or strength of the signals generated by the various light detectors, arranged according to the positions of the individual light detectors within the light detector array 104.

Cells within a fluid cause light to scatter, rather than being transmitted straight through the fluid and vessel B (along its initial path). When there are more cells in the fluid, there is more scattering of the light, with more individual light detectors receiving at least some of the light, though with a relatively low maximum intensity compared to the maximum intensity of the light received by an individual light detector when a fluid having a lower cell concentration is being analyzed. Stated differently, light passing through a fluid having a lower cell concentration will be narrowly distributed or dispersed, while light passing through a fluid having a greater cell concentration will be more widely or broadly distributed or dispersed. Thus, by providing a light detector array 104, the intensity of light received by multiple individual light detectors (i.e., the light distribution or the scattering profile) may be assessed to determine the cell concentration of a fluid.

Once the controller has generated a scattering profile, it may employ various approaches to extract data from the scattering profile that can be used to determine the cell concentration of a subject fluid. For example, U.S. Patent Application Publication No. 2023/0243746 (which is hereby incorporated by reference herein) describes how the maximum intensity or voltage and width of a portion of a scattering profile is indicative of the platelet concentration of a fluid. According to one approach described in U.S. Patent Application Publication No. 2023/0243746, the controller determines the width of a scattering profile at an intensity value equal to a particular percentage of the maximum value. Upon determining the maximum intensity or voltage and the width of the scattering profile at the selected percentage of the maximum value, the controller may determine the instantaneous platelet concentration of the fluid (e.g., by accessing a library of values correlating maximum intensities and scattering profile widths to different platelet concentration values).

According to another exemplary approach that is described in U.S. Patent Application Serial No. 18/938,376 (which is hereby incorporated by reference herein), a slope of a portion of a scattering profile may be correlated to the platelet concentration of a fluid. More particularly, a scattering profile will have an apex at the location corresponding to the individual light detector that has received the most light that has passed through the vessel B and the fluid within the vessel B. The scattering profile will have a "rising edge" to the left of the apex and a "falling edge" to the right of the apex. The rising edge encompasses signals from the individual light detectors to the left of the central light detectors, while the falling edge encompasses signals from the individual light detectors to the right of the central light detectors. As explained above, the light detectors closer to the center of the light detector array 104 will tend to receive more light than the light detectors spaced farther from the center, such that the rising edge will have a positive slope (which will tend to be different at different points along the scattering profile) and the falling edge will have a negative slope (which will tend to be different at different points along the scattering profile). It has been found that the magnitude of the rising edge slope and the falling edge slope of a scattering profile are each indicative of the platelet concentration of a fluid being monitored by an optical detection assembly such that, upon determining the rising edge slope and/or the falling edge slope of a scattering profile (by employing any suitable approach), the controller may determine the instantaneous platelet concentration of the subject fluid (e.g., by accessing a library of values correlating the rising edge slope or the falling edge slope of a scattering profile to different platelet concentration values).

It should be understood that the optical detection assembly 100 of Figs. 3-5 and the above-described platelet concentration determination techniques are merely exemplary and that differently configured optical detection assemblies (e.g., ones employing a single light detector, rather than a light detector array) and different cell concentration determination techniques may be employed without departing from the scope of the present disclosure.

With the instantaneous cell concentration values, the controller may calculate the estimated cellular yield for a procedure using the following equation:

$$Yield_{Sensor} \ = \ \Sigma(C_{Inst} \ * \ Q_{PLTs}) \text{ [Equation 1],}$$

in which

$Yield_{Sensor}$ is the estimated cellular yield for the procedure,
$C_{Inst}$ is an instantaneous cell concentration of a subject fluid (e.g., either platelet-rich plasma or platelet concentrate), and
$Q_{PLTS}$ is the flow rate of the subject fluid while a particular $C_{Inst}$ was being detected. $Q_{PLTS}$ may be determined by any suitable approach, such as by monitoring the operational rate of a pump acting to convey the subject fluid through a conduit being monitored by the optical detection assembly 100 or by monitoring a change in weight or volume of a container receiving the cells (e.g., the platelet collection container F6). It should be understood that use of Equation 1 to calculate an estimated cellular yield is merely exemplary and that other approaches to calculating the estimated cellular yield of a procedure may be employed without departing from the scope of the present disclosure.

The estimated cellular yield of a procedure may be stored in a central computer or data management system or data processing system 64 of the biological fluid processing system (Fig. 1) that communicates with the controller 18 of the biological fluid processing device 10.

V. Adjustment Of Instantaneous Cell Concentration And Estimated Cellular Yield

As noted above, after a platelet collection procedure has been completed, the collected platelet product may be analyzed to calculate an actual platelet yield. The same is true for procedures in which other blood cells are collected. In one embodiment, a collected cellular product may be analyzed using a cell counter 66 (Fig. 1) to calculate the actual cellular yield of a procedure. A cell counter of the type marketed by the Sysmex Corporation of Kobe, Japan may be employed to calculate an actual platelet yield, for example, but differently configured cell counters may also be employed without departing from the scope of the present disclosure. Additionally, other approaches to calculating the actual cellular yield of a procedure may be employed without departing from the scope of the present disclosure.

As with the estimated cellular yield, the actual cellular yield of a procedure may be stored in a donor or blood source history file of a data management system 64 of the biological fluid processing system that communicates with the controller 18 of the biological fluid processing device 10. In one embodiment, the data management system 64 may communicate with the controllers of a plurality of biological fluid processing devices (which may be identically configured to the biological fluid processing device 10 or differently configured) and/or blood donation/processing facilities. The data management system 64 may associate the estimated cellular yield and the actual cellular yield with the blood source of the procedure (e.g., a human blood donor), thus recording and storing historical data for a particular blood source that has been the subject of multiple cellular separation/collection procedures using the biological fluid processing device 10 and/or one of the other biological fluid processing devices with which the data management system 64 communicates.

As also noted above, it has been found that the estimated platelet yield calculated by a controller may differ from the actual platelet yield. This is also true for procedures in which other blood cells are collected. A cellular yield estimation error $Yield_{Sensor\text{-}Error}$ (which is also referred to herein as an "adjustment factor") may be calculated using the following equation:

$$Yield_{Sensor-Error} = \frac{Yield_{Sensor} - Yield_{Actual}}{Yield_{Sensor}} * 100 \text{ [Equation 2]},$$

in which $Yield_{Actual}$ is the actual cellular yield.

On account of $Yield_{Sensor}$ being calculated using the $C_{Inst}$ values determined by the controller 18 (and assuming that the determination of $Q_{PLTs}$ does not contain an error), it can be concluded that $Yield_{Sensor-Error}$ is due to an error in the $C_{Inst}$ measurements. Therefore, in order to improve the $Yield_{Sensor}$ value calculated by the controller 18 of a biological fluid processing device 10, the data management system 64 may provide $Yield_{Sensor-Error}$ to the controller 18 prior to the biological fluid processing device 10 being used to execute a subsequent procedure in which the same type of cells are collected for the same blood source. Alternatively, the data management system 64 may instead provide the appropriate historical data to the controller 18, with the controller 18 itself calculating the adjustment factor. With the $Yield_{Sensor-Error}$ value for that blood source, the controller 18 may carry out the cell collection procedure as usual, but adjust the determination of each $C_{Inst}$ value using the following equation:

$$C_{Inst\text{-}Adjusted} = C_{Inst} - C_{Inst} * Yield_{Sensor\text{-}Error} \text{ [Equation 3]},$$

**in which** $C_{Inst\text{-}Adjusted}$ is an adjusted instantaneous cell concentration value.

The controller 18 may then use the various $C_{Inst-Adjusted}$ values to calculate an adjusted estimated cellular yield $Yield_{Sensor-Adjusted}$ for the procedure using the following modified version of Equation 1:

$$Yield_{Sensor-Adjusted} = \sum(C_{Inst-Adjusted} * Q_{PLTs}) \text{ [Equation 4]},$$

which should result in an estimated cellular yield that is closer to the actual cellular yield of the procedure than the estimated cellular yield calculated for the previous iteration of the procedure for the same blood source. Additionally, adjusting the instantaneous cell concentration value during a procedure may result in higher efficiency collections and more cell products collected on average per procedure.

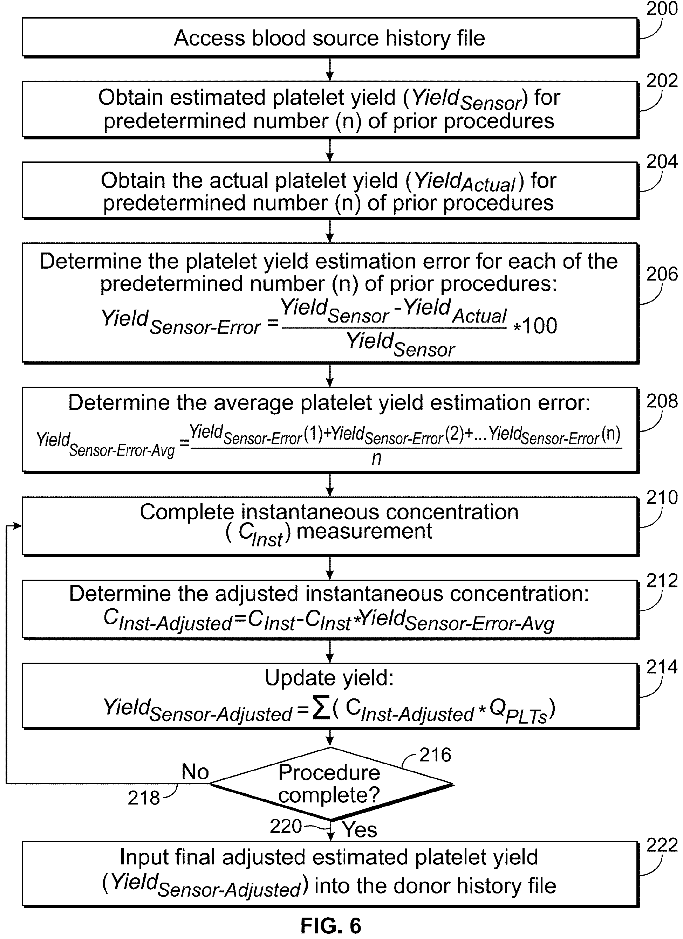

**In** the foregoing example, a cell collection procedure is only executed twice for a particular blood source, such that only one $Yield_{Sensor-Error}$ value (the one calculated for the initial iteration of the cell collection procedure) is available for adjusting the operation of the controller 18 during the later iteration of the procedure. However, when a cell collection procedure has been executed multiple times for the same blood source and the data management system 64 has stored the estimated cellular yield (or adjusted estimated cellular yield) and actual cellular yield for those procedures for that blood source, multiple $Yield_{Sensor-Error}$ values are available to be used prior to a subsequent iteration of the procedure for that blood source. Fig. 6 illustrates one exemplary approach to employing multiple historical $Yield_{Sensor-Error}$ values for a particular blood source to improve the accuracy of a $Yield_{Sensor-Adjusted}$ value produced by a controller 18 implementing a subsequent iteration of a platelet collection procedure for that same blood source. While Fig. 6 is specific to execution of a platelet collection procedure (and uses historical data from previous platelet collection procedures for the same blood source), it should be understood that the approach illustrated in Fig. 6 may be applied to the execution of procedures in which other types of cells are collected (using historical data from previous procedures in which the same type of cell was collected for the same blood source).

In step 200 of the approach illustrated in Fig. 6, the data management system 64 accesses a donor or blood source history file from its database or the controller 18 of a biological fluid processing device 10 accesses the donor or blood source history file. The estimated platelet yield (step 202) and the actual platelet yield (step 204) are accessed for each of a selected number of previous platelet collection procedures for the same blood source. If stored in the file, an adjusted estimated platelet yield for a previous platelet collection procedure may accessed from the file, rather than the estimated platelet yield being accessed for that iteration of the procedure.

The data management system 64 or the controller 18 then (in step 206) calculates $Yield_{Sensor-Error}$ for each of the selected platelet collection procedures. While Fig. 6 illustrates $Yield_{Sensor-Error}$ being calculated using the estimated platelet yield for a previous iteration of a procedure, it should be understood that the adjusted estimated platelet yield for that iteration of the procedure may instead be employed (if available).

Next, the data management system 64 or the controller 18 calculates an average adjustment factor $Yield_{Sensor-Error-Avg}$ by dividing the sum of the individual adjustment factors by the number of previous platelet collection procedures, as shown in step 208.

The controller 18 then proceeds to execute a subsequent iteration of the platelet collection procedure for the same blood source as usual, adjusting the determination of each $C_{Inst}$ value using the average adjustment factor (steps 210 and 212).

Throughout the procedure, the controller 18 continues to calculate and update the adjusted estimated platelet yield (step 214). After updating the adjusted estimated platelet yield, the controller 18 checks to determine whether the procedure is completed (step 216). If the procedure is not complete (step 218), the controller 18 will continue calculating adjusted instantaneous platelet concentrations (steps 210 and 212) and updating the adjusted estimated platelet yield (step 214). When the procedure is complete (step 220), the controller 18 will finalize the adjusted estimated platelet yield for the procedure and communicate it to the data management system 64 (step 222), where it is stored in the blood source history file. After the procedure is completed, the collected platelet product may be analyzed (e.g., using the cell counter 66) to determine the actual platelet yield, which may be communicated to the data management system 64 and stored in the blood source history file.

VI. Example

According one exemplary implementation of the principles described herein, a platelet collection procedure is to be executed for a blood source that was previously the blood source for two platelet collection procedures. In this example, the first previously executed procedure resulted in the following values:

$$Yield_{Sensor} = 6.4e11 \text{ platelets,}$$

$$Yield_{Actual} = 6.0e11 \text{ platelets,}$$

and

$$\text{Yield}_{\text{Sensor-Error}} = \frac{6.4e11 - 6.0e11}{6.4e11} * 100 = 6.25\%,$$

according to Equation 2. As described above, the first two values may be stored in a blood source history file of a data management system 64, with the third value being calculated by the data management system 64 or by the controller 18 of the biological fluid processing device 10 to be used to execute the subsequent platelet collection procedure for the blood source.

In this example, the second previously executed platelet collection procedure resulted in the following values:

$$\text{Yield}_{\text{Sensor}} = 6.2e11 \text{ platelets,}$$

$$\text{Yield}_{\text{Actual}} = 6.0e11 \text{ platelets,}$$

and

$$\text{Yield}_{\text{Sensor-Error}} = \frac{6.2e11 - 6.0e11}{6.2e11} * 100 = 3.23\%,$$

according to Equation 2. The first two values may be stored in the same blood source history file of the data management system 64 as the corresponding values for the first procedure, with the third value being calculated by the data management system 64 or by the controller 18 of the biological fluid processing device 10 to be used to execute the subsequent platelet collection procedure for the blood source.

With the adjustment factors for the two previously executed platelet collection procedures being calculated as shown above, the average adjustment factor may be calculated as follows: $\text{Yield}_{\text{Sensor-Error-Avg}} = \frac{6.25\% + 3.23\%}{2} = 4.74\%$ , according to the equation shown in step 208 of Fig. 6. As with the adjustment factors, the average adjustment factor may be calculated by the data management system 64 and transmitted to the controller 18 of the biological fluid processing device 10 to be used to execute the subsequent platelet collection procedure for the blood source or the controller 18 itself may instead calculate the average adjustment factor.

When executing the subsequent platelet collection procedure for the blood source, the controller 18 determines an instantaneous platelet concentration $C_{Inst}$ of a monitored fluid to be 1550 e3 platelets/uL at one time during the procedure. Using the equation shown in step 212 of Fig. 6, the adjusted instantaneous platelet concentration of the monitored fluid is calculated as follows:

$$C_{\text{Inst-Adjusted}} = 1550\frac{e3}{uL} - 1550\frac{e3}{uL} * 4.74\% = 1476.5e3 \text{ platelets/uL,}$$

with the controller 18 subsequently calculating the adjusted estimated platelet yield using the adjusted instantaneous platelet concentration, as described above and shown in step 214 of Fig. 6. Per step 222 of Fig. 6, the controller 18 may transmit the finalized value for the adjusted estimated platelet yield to the data management system 64 once the procedure has been completed.

VII. Aspects

Aspect 1. A biological fluid processing device comprising: a pump system; a valve system; a controller programmed to control the operation of the pump system and the valve system to execute a biological fluid processing procedure for a blood source; and an optical detection assembly comprising a light source configured and oriented to emit a light into a cell-containing fluid in a vessel during the biological fluid processing procedure for the blood source, and a light detector configured to receive at least a portion of the light exiting the vessel, wherein the controller is further programmed to receive a signal from the light detector during the biological fluid processing procedure for the blood source, with said signal from the light detector being indicative of an intensity of said at least a portion of the light received by the light detector, receive or calculate an adjustment factor derived from historical data reflecting a cell collection procedure previously executed for the blood source in which said cell was collected, and determine an adjusted cell concentration of the cell-containing fluid in the vessel during the biological fluid processing procedure for the blood source based at least in part on said signal from the light detector and said adjustment factor.

Aspect 2. The biological fluid processing device of Aspect 1, wherein the controller is further programmed to calculate an adjusted estimated cellular yield for the biological fluid processing procedure for the blood source, with said adjusted estimated cellular yield being based at least in part on said adjusted cell concentration of the cell-containing fluid in the vessel.

Aspect 3. The biological fluid processing device of any one of the preceding Aspects, wherein the adjustment factor is based at least in part on a difference between an estimated cellular yield or adjusted estimated cellular yield for the cell collection procedure previously executed for the blood source and an actual cellular yield for the cell collection procedure previously executed for the blood source.

Aspect 4. The biological fluid processing device of Aspect 3, wherein the adjustment factor reflects a percent difference between the estimated cellular yield or adjusted estimated cellular yield for the cell collection procedure previously executed for the blood source and the actual cellular yield for the cell collection procedure previously executed for the blood source.

Aspect 5. The biological fluid processing device of any one of the preceding Aspects, wherein the adjustment factor is derived from historical data reflecting a plurality of cell collection procedures previously executed for the blood source in which said cell was collected.

Aspect 6. The biological fluid processing device of Aspect 5, wherein the adjustment factor is based at least in part on an average difference between an estimated cellular yield or adjusted estimated cellular yield and an actual cellular yield for each of said plurality of cell collection procedures previously executed for the blood source in which said cell was collected.

Aspect 7. The biological fluid processing device of Aspect 6, wherein the adjustment factor reflects an average percent difference between the estimated cellular yield or adjusted estimated cellular yield and the actual cellular yield for each of said plurality of cell collection procedures previously executed for the blood source in which said cell was collected.

Aspect 8. The biological fluid processing device of any one of the preceding Aspects, wherein said cell is a platelet and said adjusted cell concentration is an adjusted platelet concentration.

Aspect 9. A biological fluid processing system comprising: a biological fluid processing device including a pump system, a valve system, a controller programmed to control the operation of the pump system and the valve system to execute a biological fluid processing procedure for a blood source, and an optical detection assembly comprising a light source configured and oriented to emit a light into a cell-containing fluid in a vessel during the biological fluid processing procedure for the blood source, and a light detector configured to receive at least a portion of the light exiting the vessel; and a data management system programmed to store historical data reflecting a cell collection procedure previously executed for the blood source in which said cell was collected, wherein the controller is further programmed to receive a signal from the light detector during the biological fluid processing procedure for the blood source, with said signal from the light detector being indicative of an intensity of said at least a portion of the light received by the light detector, receive from the data management system an adjustment factor derived from said historical data or receive said historical data from the data management system and calculate said adjustment factor, and determine an adjusted cell concentration of the cell-containing fluid in the vessel during the biological fluid processing procedure for the blood source based at least in part on said signal from the light detector and said adjustment factor.

Aspect 10. The biological fluid processing system of Aspect 9, wherein the controller is further programmed to calculate an adjusted estimated cellular yield for the biological fluid processing procedure for the blood source, with said adjusted estimated cellular yield being based at least in part on said adjusted cell concentration of the cell-containing fluid in the vessel.

Aspect 11. The biological fluid processing system of any one of Aspects 9-10, wherein the adjustment factor is based at least in part on a difference between an estimated cellular yield or adjusted estimated cellular yield for the cell collection procedure previously executed for the blood source and an actual cellular yield for the cell collection procedure previously executed for the blood source.

Aspect 12. The biological fluid processing system of Aspect 11, wherein the adjustment factor reflects a percent difference between the estimated cellular yield or adjusted estimated cellular yield for the cell collection procedure previously executed for the blood source and the actual cellular yield for the cell collection procedure previously executed for the blood source.

Aspect 13. The biological fluid processing system of any one of Aspects 9-12, wherein the adjustment factor is derived from historical data reflecting a plurality of cell collection procedures previously executed for the blood source in which said cell was collected.

Aspect 14. The biological fluid processing system of Aspect 13, wherein the adjustment factor is based at least in part on an average difference between an estimated cellular yield or adjusted estimated cellular yield and an actual cellular yield for each of said plurality of cell collection procedures previously executed for the blood source in which said cell was collected.

Aspect 15. The biological fluid processing system of Aspect 14, wherein the adjustment factor reflects an average percent difference between the estimated cellular yield or adjusted estimated cellular yield and the actual cellular yield for each of said plurality of cell collection procedures previously executed for the blood source in which said cell was collected.

Aspect 16. The biological fluid processing system of any one of Aspects 9-15, further comprising a cell counter configured to determine an actual cellular yield for the biological fluid processing procedure for the blood source, wherein the data management system is programmed to store the actual cellular yield for the biological fluid processing procedure for the blood source.

Aspect 17. The biological fluid processing system of any one of Aspects 9-16, wherein said cell is a platelet and said adjusted cell concentration is an adjusted platelet concentration.

Aspect 18. A controller-implemented method of executing a biological fluid processing procedure for a blood source, the method comprising: separating a cell-containing fluid from blood from the blood source; emitting light through the cell-containing fluid; receiving at least a portion of the light exiting the cell-containing fluid; generating a signal indicative of an intensity of said at least a portion of the light exiting the cell-containing fluid; receiving or calculating an adjustment factor derived from historical data reflecting a cell collection procedure previously executed for the blood source in which said cell was collected; and determining an adjusted cell concentration of the cell-containing fluid based at least in part on said signal and said adjustment factor.

Aspect 19. The method of Aspect 18, further comprising calculating an adjusted estimated cellular yield for the biological fluid processing procedure for the blood source, with said adjusted estimated cellular yield being based at least in part on said adjusted cell concentration of the cell-containing fluid.

Aspect 20. The method of any one of Aspects 18-19, wherein the adjustment factor is based at least in part on a difference between an estimated cellular yield or adjusted estimated cellular yield for the cell collection procedure previously executed for the blood source and an actual cellular yield for the cell collection procedure previously executed for the blood source.

Aspect 21. The method of Aspect 20, wherein the adjustment factor reflects a percent difference between the estimated cellular yield or adjusted estimated cellular yield for the cell collection procedure previously executed for the blood source and the actual cellular yield for the cell collection procedure previously executed for the blood source.

Aspect 22. The method of any one of Aspects 18-21, wherein the adjustment factor is derived from historical data reflecting a plurality of cell collection procedures previously executed for the blood source in which said cell was collected.

Aspect 23. The method of Aspect 22, wherein the adjustment factor is based at least in part on an average difference between an estimated cellular yield or adjusted estimated cellular yield and an actual cellular yield for each of said plurality of cell collection procedures previously executed for the blood source in which said cell was collected.

Aspect 24. The method of Aspect 23, wherein the adjustment factor reflects an average percent difference between the estimated cellular yield or adjusted estimated cellular yield and the actual cellular yield for each of said plurality of cell collection procedures previously executed for the blood source in which said cell was collected.

Aspect 25. The method of any one of Aspects 18-24, wherein said cell is a platelet and said adjusted cell concentration is an adjusted platelet concentration.

It will be understood that the embodiments described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A biological fluid processing device (10) comprising:

   a pump system (P1-P6);
   a valve system (V1-V9);
   a controller (18) programmed to control the operation of the pump system (P1-P6) and the valve system (V1-V9) to execute a biological fluid processing procedure for a blood source; and
   an optical detection assembly (100) comprising

a light source (102) configured and oriented to emit a light into a cell-containing fluid in a vessel during the biological fluid processing procedure for the blood source, and

a light detector (104) configured to receive at least a portion of the light exiting the vessel, wherein the controller (18) is further programmed to

receive a signal from the light detector (104) during the biological fluid processing procedure for the blood source, with said signal from the light detector (104) being indicative of an intensity of said at least a portion of the light received by the light detector (104),

receive or calculate an adjustment factor derived from historical data reflecting a cell collection procedure previously executed for the blood source in which said cell was collected, and

determine an adjusted cell concentration of the cell-containing fluid in the vessel during the biological fluid processing procedure for the blood source based at least in part on said signal from the light detector (104) and said adjustment factor.

2. A biological fluid processing system comprising:

the biological fluid processing device (10) of claim 1; and

a data management system (64) programmed to store said historical data, wherein the controller (18) is further programmed to receive said adjustment factor from the data management system (64) or receive said historical data from the data management system (64) and calculate said adjustment factor.

3. The biological fluid processing device (10) of claim 1 or the biological fluid processing system of claim 2, wherein the controller (18) is further programmed to calculate an adjusted estimated cellular yield for the biological fluid processing procedure for the blood source, with said adjusted estimated cellular yield being based at least in part on said adjusted cell concentration of the cell-containing fluid in the vessel.

4. The biological fluid processing device (10) of any one of claims 1 and 3 or the biological fluid processing system of any one of claims 2 and 3, wherein the adjustment factor is based at least in part on a difference between an estimated cellular yield or adjusted estimated cellular yield for the cell collection procedure previously executed for the blood source and an actual cellular yield for the cell collection procedure previously executed for the blood source.

5. The biological fluid processing device (10) or the biological fluid processing system of claim 4, wherein the adjustment factor reflects a percent difference between the estimated cellular yield or adjusted estimated cellular yield for the cell collection procedure previously executed for the blood source and the actual cellular yield for the cell collection procedure previously executed for the blood source.

6. The biological fluid processing device (10) of any one of claims 1 and 3-5 or the biological fluid processing system of any one of claims 2-5, wherein the adjustment factor is derived from historical data reflecting a plurality of cell collection procedures previously executed for the blood source in which said cell was collected.

7. The biological fluid processing device (10) or the biological fluid processing system of claim 6, wherein the adjustment factor is based at least in part on an average difference between an estimated cellular yield or adjusted estimated cellular yield and an actual cellular yield for each of said plurality of cell collection procedures previously executed for the blood source in which said cell was collected.

8. The biological fluid processing device (10) or the biological fluid processing system of claim 7, wherein the adjustment factor reflects an average percent difference between the estimated cellular yield or adjusted estimated cellular yield and the actual cellular yield for each of said plurality of cell collection procedures previously executed for the blood source in which said cell was collected.

9. The biological fluid processing system of any one of claims 2-8, further comprising a cell counter (66) configured to determine an actual cellular yield for the biological fluid processing procedure for the blood source, wherein the data management system (64) is programmed to store the actual cellular yield for the biological fluid processing procedure for the blood source.

10. A controller-implemented method of executing a biological fluid processing procedure for a blood source, the method comprising:

separating a cell-containing fluid from blood from the blood source;

emitting light through the cell-containing fluid;
receiving at least a portion of the light exiting the cell-containing fluid;
generating a signal indicative of an intensity of said at least a portion of the light exiting the cell-containing fluid;
receiving or calculating an adjustment factor derived from historical data reflecting a cell collection procedure previously executed for the blood source in which said cell was collected; and
determining an adjusted cell concentration of the cell-containing fluid based at least in part on said signal and said adjustment factor.

11. The method of claim 10, further comprising calculating an adjusted estimated cellular yield for the biological fluid processing procedure for the blood source, with said adjusted estimated cellular yield being based at least in part on said adjusted cell concentration of the cell-containing fluid.

12. The method of any one of claims 10-11, wherein the adjustment factor is based at least in part on a difference between an estimated cellular yield or adjusted estimated cellular yield for the cell collection procedure previously executed for the blood source and an actual cellular yield for the cell collection procedure previously executed for the blood source.

13. The method of claim 12, wherein the adjustment factor reflects a percent difference between the estimated cellular yield or adjusted estimated cellular yield for the cell collection procedure previously executed for the blood source and the actual cellular yield for the cell collection procedure previously executed for the blood source.

14. The method of any one of claims 10-13, wherein the adjustment factor is derived from historical data reflecting a plurality of cell collection procedures previously executed for the blood source in which said cell was collected.

15. The method of claim 14, wherein the adjustment factor is based at least in part on an average difference between an estimated cellular yield or adjusted estimated cellular yield and an actual cellular yield for each of said plurality of cell collection procedures previously executed for the blood source in which said cell was collected.

**FIG. 1**

**FIG. 2**

EP 4 763 245 A1

**FIG. 3**

**FIG. 4**

**FIG. 5**

Access blood source history file — 200

Obtain estimated platelet yield ($Yield_{Sensor}$) for predetermined number (n) of prior procedures — 202

Obtain the actual platelet yield ($Yield_{Actual}$) for predetermined number (n) of prior procedures — 204

Determine the platelet yield estimation error for each of the predetermined number (n) of prior procedures:
$$Yield_{Sensor\text{-}Error} = \frac{Yield_{Sensor} - Yield_{Actual}}{Yield_{Sensor}} * 100$$
— 206

Determine the average platelet yield estimation error:
$$Yield_{Sensor\text{-}Error\text{-}Avg} = \frac{Yield_{Sensor\text{-}Error}(1) + Yield_{Sensor\text{-}Error}(2) + \ldots Yield_{Sensor\text{-}Error}(n)}{n}$$
— 208

Complete instantaneous concentration ($C_{Inst}$) measurement — 210

Determine the adjusted instantaneous concentration:
$$C_{Inst\text{-}Adjusted} = C_{Inst} - C_{Inst} * Yield_{Sensor\text{-}Error\text{-}Avg}$$
— 212

Update yield:
$$Yield_{Sensor\text{-}Adjusted} = \sum (C_{Inst\text{-}Adjusted} * Q_{PLTs})$$
— 214

Procedure complete? — 216

No — 218

Yes — 220

Input final adjusted estimated platelet yield ($Yield_{Sensor\text{-}Adjusted}$) into the donor history file — 222

**FIG. 6**

EP 4 763 245 A1

Europäisches Patentamt
European Patent Office
Office européen des brevets

# EUROPEAN SEARCH REPORT

**Application Number**

EP 25 22 4875

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/131334 A1 (LANGLEY ROBERT W [US] ET AL) 16 June 2005 (2005-06-16)<br>* paragraph [0003] - paragraph [0012] *<br>* paragraph [0030] *<br>* paragraph [0038] *<br>* paragraph [0041] - paragraph [0066] *<br>* figures 1,3,4 *<br>----- | 1-15 | INV.<br>A61M1/36<br>A61M1/38<br>G01N33/49 |
| X<br><br>A | EP 1 065 495 A2 (TERUMO CORP [JP]) 3 January 2001 (2001-01-03)<br>* paragraph [0004] - paragraph [0007] *<br>* paragraph [0034] - paragraph [0037] *<br>* paragraph [0053] - paragraph [0063] *<br>* paragraph [0066] *<br>* figures 1,2,4 *<br>----- | 1-3,6,<br>10,11,14<br>4,5,7-9,<br>12,13,15 | |

**TECHNICAL FIELDS SEARCHED (IPC)**

A61M
G01N

The present search report has been drawn up for all claims

| Place of search | Date of completion of the search | Examiner |
|---|---|---|
| The Hague | 25 April 2026 | Kempeneers, Johanna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

24

**EP 4 763 245 A1**

## ANNEX TO THE EUROPEAN SEARCH REPORT
## ON EUROPEAN PATENT APPLICATION NO.

EP 25 22 4875

25-04-2026

| Patent document cited in search report | | Publication date | Patent family member(s) | | Publication date |
|---|---|---|---|---|---|
| US 2005131334 | A1 | 16-06-2005 | CA | 2100199 A1 | 11-01-1994 |
| | | | DE | 69313212 T2 | 02-01-1998 |
| | | | EP | 0580299 A1 | 26-01-1994 |
| | | | JP | 2907689 B2 | 21-06-1999 |
| | | | JP | H06335524 A | 06-12-1994 |
| | | | US | 5605842 A | 25-02-1997 |
| | | | US | 5611997 A | 18-03-1997 |
| | | | US | 6319471 B1 | 20-11-2001 |
| | | | US | 2002046975 A1 | 25-04-2002 |
| | | | US | 2004096814 A1 | 20-05-2004 |
| | | | US | 2005131334 A1 | 16-06-2005 |
| EP 1065495 | A2 | 03-01-2001 | AT | E317113 T1 | 15-02-2006 |
| | | | DE | 60025774 T2 | 12-10-2006 |
| | | | EP | 1065495 A2 | 03-01-2001 |
| | | | JP | 3817091 B2 | 30-08-2006 |
| | | | JP | 2001021479 A | 26-01-2001 |
| | | | US | 6678040 B1 | 13-01-2004 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5194145 A
- WO 2018053217 A1
- US 5868696 A
- US 6419822 B
- US 8556793 B
- US 20230243746
- US 938376